Europäisches Patentamt

European Patent Office

Office européen des brevets

(11) Publication number: **0 420 605 A1**

## EUROPEAN PATENT APPLICATION

(21) Application number: 90310521.1

(51) Int. Cl.⁵: **A61B 17/06**

(22) Date of filing: 26.09.90

(30) Priority: 27.09.89 US 413240
04.06.90 US 532928
05.09.90 US 576046

(43) Date of publication of application:
**03.04.91 Bulletin 91/14**

(84) Designated Contracting States:
**CH DE ES FR GB IT LI NL SE**

(71) Applicant: **UNITED STATES SURGICAL
CORPORATION
150 Glover Avenue
Norwalk, Connecticut 06856(US)**

(72) Inventor: **Korthoff, Herbert W.
22 Cross Way
Westport CT 06880(US)**
Inventor: **Granger, Richard N.
18 Arrowhead Lane
Huntingdon, CT 06484(US)**

(74) Representative: **Marsh, Roy David et al
Urquhart-Dykes & Lord Midsummer House
411C Midsummer Boulevard
Central Milton Keynes MK9 3BN(GB)**

(54) Combined surgical needle-suture device and method for its manufacture.

(57) A suture (30) is attached to a surgical needle (10) possessing a shank (12) of reduced diameter. Attachment is accomplished by means of a shrinkable tubing (20) which is fitted about the shank of the needle and an end of the suture, application of heat to the tubing effecting its shrinkage with consequent secure attachment of the needle to the suture.

FIG. 1

Xerox Copy Centre

## COMBINED SURGICAL NEEDLE-SUTURE DEVICE AND METHOD FOR ITS MANUFACTURE

### BACKGROUND OF THE INVENTION

The present invention relates to a combined surgical needle-suture device, and, more particularly, to such a device in which a shrinkable tubing attaches a suture to a surgical needle.

For many years, surgeons have employed needle-suture combinations in which a suture or ligature is attached to the shank end of a needle. Such needle-suture combinations are provided for a wide variety of monofilament and braided suture materials, both absorbable and non-absorbable, e.g., catgut, silk, nylon, polyester, polypropylene, linen, cotton, and absorbable synthetic materials such as polymers and copolymers of glycolic and lactic acids.

Needle-suture combinations fall into two general classes: standard needle attachment and removable or detachable needle attachment. In the case of standard needle attachment, the suture is securely attached to the needle and is not intended to be separable therefrom, except by cutting or severing the suture. Removable needle attachment, by contrast, is such that the needle is separable from the suture in response to a force exerted by the surgeon. Minimum acceptable forces required to separate a needle from a suture (for various suture sizes) are set forth in the United States Pharmacopeia (USP). The USP prescribes minimum individual pull-out forces and minimum average pull-out forces as measured for five needle-suture combinations. The minimum pull-out forces for both standard and removable needle-suture attachment set forth in the USP are hereby incorporated by reference.

One typical method for securing a suture to a needle involves providing a cylindrical recess in the shank end of a needle and securing a suture therein. For example, U.S. Patent No. 1,558,037 teaches the addition of a cement material to such a substantially cylindrical recess to secure the suture therein. Additional methods for bonding a suture within a needle bore are described in U.S. Patent Nos. 2,928,395 (adhesives) and 3,394,704 (bonding agents). Alternatively, a suture may be secured within an axial bore in a needle by swaging the needle in the region of the recess. See, e.g., U.S. Patent No. 1,250,114. Additional prior art methods for securing a suture within a needle bore include expansion of a catgut suture through the application of heat (U.S. Patent No. 1,665,216), inclusion of protruding teeth within the axial bore to grasp an inserted suture (U.S. Patent No. 1,678,361) and knotting the end of the suture to be inserted within the bore to secure the suture therein (U.S. Patent No. 1,757,129).

Methods for detachably securing a suture to a needle are also well known. For example, U.S. Patent Nos. 3,890,975 and 3,980,177 teach swaging a suture within a needle bore such that the suture has a pull-out value of 3 to 26 ounces. Alternative detachable attachment methods include providing a weakened suture segment (U.S. Patent No. 3,949,756), lubricant tipping the end of a suture to be inserted in the axial bore of a needle (U.S. Patent No. 3,963,031), and pre-tensioning a suture that is swaged within an axial needle bore (U.S. Patent No. 3,875,946). See also, U.S. Patent Nos. 3,799,169; 3,880,167; 3,924,630; 3,926,194; 3,943,933; 3,981,307; 4,124,027; and, 4,127,133.

Another method for attaching a suture to a needle involves the use of tubing which is secured to the shank end of the needle and to the suture. For example, U.S. Patent No. 1,613,206 describes the use of a tubing (preferably silver) which is secured to the shank end of a needle and to a ligature. It is suggested that the tube may be attached to the needle by pressure or soldering and to the ligature by pressure or cementing. It is also suggested that the shank of the needle be of reduced cross section and that the furthest extremity of the reduced diameter shank section be provided with a spike or point upon which the suture may be secured prior to tube application.

U.S. Patent No. 2,240,330 describes a tubing attachment method whereby the tubing and suture are releasably secured to the needle. In particular, the needle and tubing are provided with cooperating catch and abutment means which are released one from the other by rotating the needle 90° relative to the tubing (or vice versa). The tubing is manufactured from spring-tempered carbon steel or chrome nickel steel and is secured to the suture by heating the tubing and then swaging to the suture.

U.S. Patent No. 3,311,100 relates to a flexible composite suture having a tandem linkage. The needle is secured to a flexible suture leader manufactured from a readily sterilizable plastic such as nylon, linear polyethylene, isotactic polypropylene, polyester, silk or other proteinaceous material, e.g. by inserting and crimping the leader within an axial bore in the needle shank. The opposite end of the suture leader is crimped within a connector sleeve of a thin walled metal tubing, e.g., stainless steel. The opposite end of the tubing is crimped around a stiff suture, e.g., monofilament stainless steel.

U.S. Patent No. 3,918,455 describes a needle-suture attachment wherein a hollow suture portion is secured to the shank end of a needle which is of reduced cross-section as compared to the remainder of the needle.

Additional patents which describe the use of tubing to effect suture-needle attachment include U.S. Patent Nos. 4,672,734 (forming needle from U-shaped metal plate around suture), 4,359,053 (silicone tubing), 3,835,912 (laser welding of metal tube to needle), 2,814,296, 2,802,468 (chamfered tubing ends), 2,302,986, 2,240,330, 1,981,651 (needle and tubing screw threaded), 1,960,117, and 1,591,021.

Numerous disadvantages exist with methods used heretofore to effect needle-suture attachment. For example, those methods which involve the formation and use of an axial bore in the shank end of the needle require the use of expensive hole forming equipment. Moreover, it is difficult to maintain the bore concentric with the center-line of the needle and to control the depth (and diameter) of the bore when drilling the needle shank, whether using conventional drilling equipment or laser drilling. Another disadvantage is the possibility that foreign substances may inadvertently or uncontrollably be introduced into the needle bore, e.g., oil used during drilling or silicone from the needle silconization process. Safeguards employed in an attempt to prevent the introduction of such foreign materials, e.g., water blocking during needle silconization, are inconvenient adding time, effort and cost to the needle production process.

Attachment processes which employ bored needle shanks also limit the range of materials from which needles may be fabricated in a cost effective fashion. For example, it is exceedingly difficult to drill Series 300 stainless steel (laser drilling is required) and, once drilled, it is difficult to swage Series 300 stainless steel in a consistent and reliable manner. For this reason, Series 300 stainless steel is not employed for the vast majority of needled suture products despite its advantageous combination of strength and ductility characteristics as compared to conventionally employed Series 400 stainless steel.

Additional disadvantages associated with needle-suture attachment methods which employ bored needle shanks include the weakness imparted to the bored section of the needle, particularly after swaging, and the attendant increased possibility that the needle will fracture in this region. It is also difficult to provide a specialized surface finish to the needle shank to assist in needle attachment, e.g., a texturized surface and/or a tapered bore. Swaging equipment used in such needle-suture attachment methods is also maintenance intensive.

Needle-suture attachment methods which have employed tubings heretofore also exhibit numerous disadvantages. Methods which employ metal tubings greatly diminish the flexibility of the needle-suture combination in the attachment region. Such diminished flexibility has a deleterious effect in many surgical procedures. Swaging of the tubing to the needle and the suture is also undesirable in that swaging is time-consuming, maintenance intensive, and subject to variability in attachment force.

Moreover, needle-suture attachment methods which have employed tubings heretofore have necessarily required the use of tubing having an inner diameter essentially equal to the outer diameters of the needle shank and suture tip to be attached. Too large a difference between the aforesaid inner and outer diameters inhibits the attachment process, and prevents a tight, secure interface between needle (and/or suture) and tubing. The limited tolerance between the tubing inner diameter and the needle shank/suture outer diameters in such methods make these dimensions critical, thereby making the attachment process more difficult and time-consuming, and increasing the likelihood of attachment failure and/or rejected materials.

Accordingly, it is an object of the present invention to provide a needle-suture attachment means which eliminates the need for drilling a bore in the needle shank and swaging a suture therein.

It is also an object of the invention to provide an attachment means which reliably secures the suture to the needle in an efficient manner while also preserving the integrity of the suture material during surgical procedures.

It is a further object of the present invention to provide a needle-suture attachment which is flexible and atraumatic.

An additional object of the invention is to provide a combined needle-suture device that can be non-detachable or detachable in form.

## SUMMARY OF THE INVENTION

The present invention provides a combined surgical needle-suture device which comprises:
a) a needle having a shank of reduced cross-section;
b) a suture; and,
c) a shrinkable tubing around said needle shank and a portion of said suture.

The combined surgical needle-suture device is manufactured by the method which comprises:
a) providing a needle having a shank of reduced diameter;
b) placing a shrinkable tubing around the reduced diameter needle shank and a suture to be attached thereto; and

c) applying energy to the shrinkable tubing to bring the tubing into engagement with the needle shank and the suture.

The physical and chemical characteristics of the shrinkable tubing material, the relative diameters of the tubing, the needle shank and the suture, and the amount of energy applied to the tubing may be controlled to provide a needle-suture combination having a desired pull-out force. It is thus possible to produce standard needle-suture combinations and removable needle-suture combinations using a single attachment process and a common inventory of materials.

BRIEF DESCRIPTION OF THE DRAWINGS

The present invention will become more apparent from the following detailed description taken in conjunction with the accompanying drawings in which:

Figure 1 is a side cross-sectional view of a needle and a suture with a tubing positioned therearound (prior to engagement of the tubing with the needle and suture);

Figure 2 is a side cross-sectional view of the tubing of Figure 1 in engagement with the needle and suture; and

Figure 3 is a side view of a needle-suture attachment of the present invention.

DETAILED DESCRIPTION OF THE INVENTION

The present invention relates to a combined surgical needle-suture device and surgical needle-suture attachment method. The invention has application to any suture material whether absorbable or non-absorbable, natural or synthetic, braided or monofilament, and to any needle material and configuration whether straight or curved. The invention may be used to effect standard or detachable needle attachment as described in more detail below.

The butt end of the reduced diameter shank of the combined needle-suture device may be flat or rounded, preferably the latter. When the butt end of the needle shank is rounded, a surgeon can more readily "turn corners" and/or flex the butt end during the suturing procedure without incurring a risk that the butt end of the shank will protrude through or up the attached tubing. Accordingly, providing the butt end of the shank with rounded corners or edges enhances the security and reliability of the needle-suture attachment and diminishes the possibility of premature, unintended detachment of the needle from its suture during a suturing procedure.

Another embodiment of the present invention makes use of the relationship between the length of the tubing and the effective axial length of the needle shank to reliably achieve standard (i.e., ordinarily non-detachable) or selectively reversible (i.e., detachable) attachment of the needle and suture in the combined surgical needle-suture devices of the present invention. The expression "effective axial length of the needle shank" shall be understood to include only so much of the length of the needle shank which is actually in contact with the interior wall of the tubing and excludes any portion of length of the needle shank which does not make contact with the interior wall of the tubing when the needle and its attached suture are aligned along a common axis. In accordance with this embodiment of the combined surgical needle-suture device herein, for an entire line of surgical needles differing in length, diameter and configuration, attachment of the needles to their sutures can be accomplished with a minimum of two sizes of tubing, the tubing of greater axial length being used for suture-needle devices of the non-detachable variety and the tubing of shorter axial length being used for the suture-needle devices of the detachable variety. The use of such tubings makes it possible to minimize the number of different tubings which must be manufactured and inventoried to accommodate the needle-to-suture attachment needs of a comprehensive line of combined needle-suture devices.

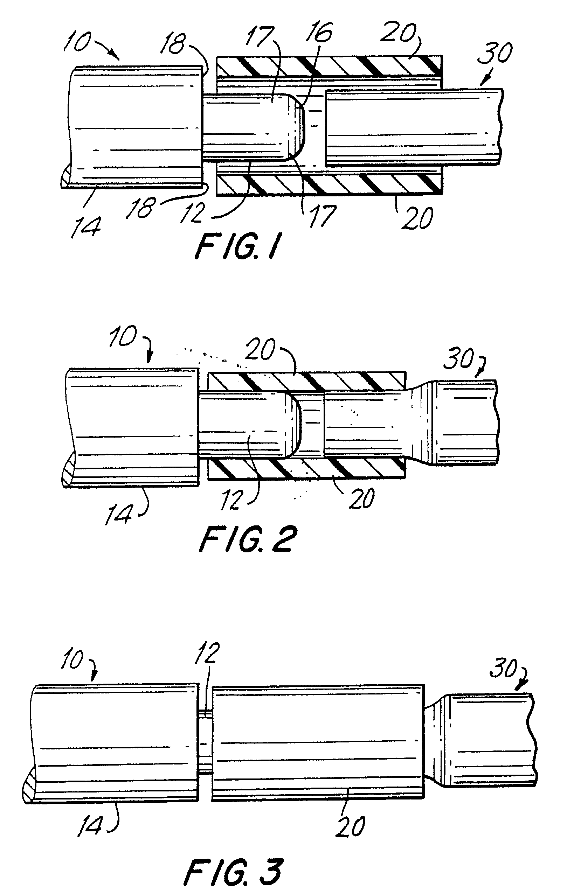

Referring to Figs. 1-3, needle 10 has a reduced cross-sectional diameter at its shank end 12 relative to the remaining portion 14 of the needle. The diameter of shank end 12 may be reduced by any conventional means, e.g., by turning on a lathe. Typically, shank end 12 has a diameter from 10 to 65% smaller than the remainder of the needle 14, and preferably from 25 to 50% smaller. It is also possible to provide shank end 12 with a texturized surface to facilitate gripping by shrinkable tubing 20. For example, shank end 12 may be scored, ribbed or threaded, in whole or in part. It may also be desirable to taper shank end 12 such that its butt, or distal end 16 is of greater cross-sectional diameter than the cross-sectional diameter of shank end 12 in the region of shoulder 18, or vice versa .

4

As stated above, butt end i6 advantageously possesses rounded, beveled or chamfered force-distributing corners, or edges, 17 which serve to prevent shank end 12 from protruding through or ripping shrinkable tubing 20 in the attached configuration of the combined surgical needle-suture device as illustrated in Fig. 2. Bevelling or chamfering of the edges of butt end 16, while marginally less effective than rounding, are to be considered equivalent to the latter for the purpose of this invention. Accordingly, as used herein, the terms "rounding" and "rounded" shall be taken as essentially equivalent to "beveling", "beveled", "chamfering" and "chamfered". As the radius of curvature of rounded corners 17 increases to some maximum value, e.g., that of the radius of butt end 16 when in cross section the butt end defines a circle, curved corners 17 will approach a hemispherical shape which maximizes the distribution of the force with which tubing 20 is made to bear against the edges of shank 12 when the suture is brought under tension against its attached needle at some sharp angle. However, even with a radius of curvature which is less than the maximum achievable for a particular needle shank, e.g., 20-50% of the maximum radius of curvature, rounded corners 17 will still tend to prevent excessive distortion of, or damage to, tubing 20 although possibly with less effectiveness in this regard.

Rounding, beveling or chamfering of the butt end 16 of shank 12 to provide force-distributing corners 17 can be accomplished by any of a variety of conventional machining techniques, e.g., grinding, lathing, tumbling, etc.

Suture 30 is also positioned within shrinkable tubing, or micro-ferrule, 20. When, as shown in Figs. 1-3 a gap exists between end 21 of tubing 20 and shoulder 18 of the needle, i.e., tubing 20 extends for less than the full length of needle shank 12, the effective axial length of shank 12 will correspond only to that length of the shank which is actually in contact with the interior surface of the tubing. However, when the end 21 of tubing 20 abuts shoulder 18 of the needle (not shown), the effective axial length of shank 12 will be equivalent to its entire length.

When suture-needle device 10 is to be of the non-detachable variety, the ratio of the axial length of tubing 20 to the effective axial length of needle shank 12 should be at least about 4:1 and is preferably at least about 4.5:1. For practical reasons, it is preferable that this ratio not exceed about 12:1 and better yet, that it not exceed about 9.5:1. For the non-detachable variety of surgical needle-suture attachment, the material of construction of tubing 20 should be such that the tubing will withstand at least the USP minimum pull-out force for the appropriate suture size. If necessary or desirable, tubing 20 can be provided with a reinforcement, e.g., of carbon fibers, glass fibers, etc., to increase its tensile strength and thus improve the ability of the tubing to withstand the stress of a relatively high pull-out force.

When suture-needle device 10 is to be of the detachable variety, the ratio of the axial length of tubing 20 to the effective axial length of needle shank 12 must not exceed about 3.5:1, and preferably should not exceed about 3.2:1. Again, for practical considerations, it is preferable that this ratio be not less than about 1.2:1 and still more preferable that it be not less than about 1.4:1.

In Tables I and II below, specific ratios of the axial length of the tubing (ALT) to the effective axial length of the shank (EALS) are given for a variety of non-detachable and detachable combined surgical needle-suture devices manufactured from different types of sutures and needles. In these tables, the designations of the suture type have the following meanings:

BRS - Braided Silk
BRD - Braided Dacron
BSA - Braided Synthetic Absorbable
BRN - Braided Nylon
PPM - Polypropylene Monofilament
SGC - Chromic Gut

TABLE I: NON-DETACHABLE SUTURES

| Suture Type | Needle Size | Needle Diameter/ Inch | Shank Diameter/ Inch | EALS/ Inch | ALT/ Inch | Ratio of ALT to EALS |
|---|---|---|---|---|---|---|
| BSA | 3/0 | .024 | .010 | .055 | .3-.5 | 5.5:1-9.1:1 |
| BRD | 3/0 | .024 | .010 | .055 | .3-.5 | 5.5:1-9.1:1 |
| BRS | 3/0 | .024 | .010 | .055 | .3-.5 | 5.5:1-9.1:1 |
| BRN | 3/0 | .024 | .010 | .055 | .3-.5 | 5.5:1-9.1:1 |
| BRN | 3/0 | .032 | .010 | .055 | .3-.5 | 5.5:1-9.1:1 |
| BRS | 4/0 | .032 | .010 | .055 | .3-.5 | 5.5:1-9.1:1 |
| BRS | 3/0 | .032 | .010 | .055 | .3-.5 | 5.5:1-9.1:1 |
| BRN | 4/0 | .032 | .010 | .055 | .3-.5 | 5.5:1-9.1:1 |
| BSA | 2/0 | .026 | .016 | .065 | .3-.5 | 4.6:1-7.7:1 |
| BRD | 2/0 | .026 | .016 | .065 | .3-.5 | 4.6:1-7.7:1 |
| BRS | 2/0 | .026 | .016 | .065 | .3-.5 | 4.6:1-7.7:1 |
| BRN | 2/0 | .026 | .016 | .065 | .3-.5 | 4.6:1-7.7:1 |
| SGC | 0 | .044 | .016 | .065 | .3-.5 | 4.6:1-7.7:1 |
| BSA | 0 | .044 | .016 | .065 | .3-.5 | 4.6:1-7.7:1 |
| SGC | 1 | .044 | .020 | .070 | .3-.5 | 4.3:1-7.1:1 |
| BSA | 1 | .044 | .020 | .070 | .3-.5 | 4.3:1-7.1:1 |
| BRD | 1 | .050 | .020 | .070 | .3-.5 | 4.3:1-7.1:1 |
| BRN | 1 | .050 | .020 | .070 | .3-.5 | 4.3:1-7.1:1 |
| BSA | 1 | .050 | .020 | .070 | .3-.5 | 4.3:1-7.1:1 |
| BRN | 1 | .039 | .020 | .070 | .3-.5 | 4.3:1-7.1:1 |
| SGC | 1 | .039 | .020 | .070 | .3-.5 | 4.3:1-7.1:1 |
| BRS | 3/0 | .039 | .010 | .055 | .3-.5 | 5.5:1-9.1:1 |
| BSA | 3/0 | .039 | .010 | .055 | .3-.5 | 5.5:1-9.1:1 |
| PPM | 2/0 | .039 | .016 | .065 | .3-.5 | 4.6:1-7.7:1 |
| BRS | 0 | .039 | .016 | .065 | .3-.5 | 4.6:1-7.7:1 |
| BRD | 2/0 | .039 | .016 | .065 | .3-.5 | 4.6:1-7.7:1 |
| PPM | 0 | .039 | .016 | .065 | .3-.5 | 4.6:1-7.7:1 |
| BRN | 2/0 | .039 | .016 | .065 | .3-.5 | 4.6:1-7.7:1 |
| BRD | 0 | .039 | .016 | .065 | .3-.5 | 4.6:1-7.7:1 |
| BSA | 2/0 | .039 | .016 | .065 | .3-.5 | 4.6:1-7.7:1 |
| SGC | 0 | .039 | .016 | .065 | .3-.5 | 4.6:1-7.7:1 |

TABLE I: NON-DETACHABLE SUTURES
(continued)

| Suture Type | Needle Size | Needle Diameter/ Inch | Shank Diameter/ Inch | EALS/ Inch | ALT/ Inch | Ratio of ALT to EALS |
|---|---|---|---|---|---|---|
| BRN | 0 | .039 | .016 | .065 | .3-.5 | 4.6:1-7.7:1 |
| BSA | 0 | .039 | .016 | .065 | .3-.5 | 4.6:1-7.7:1 |
| BRS | 2/0 | .039 | .016 | .065 | .3-.5 | 4.6:1-7.7:1 |
| BSA | 0 | .050 | .016 | .065 | .3-.5 | 4.6:1-7.7:1 |
| BRD | 0 | .050 | .016 | .065 | .3-.5 | 4.6:1-7.7:1 |
| BRN | 0 | .050 | .016 | .065 | .3-.5 | 4.6:1-7.7:1 |
| PPM | 0 | .050 | .016 | .065 | .3-.5 | 4.6:1-7.7:1 |
| PPM | 0 | .039 | .016 | .065 | .3-.5 | 4.6:1-7.7:1 |
| BRD | 0 | .039 | .016 | .065 | .3-.5 | 4.6:1-7.7:1 |
| BRN | 0 | .039 | .016 | .065 | .3-.5 | 4.6:1-7.7:1 |
| BSA | 2/0 | .039 | .016 | .065 | .3-.5 | 4.6:1-7.7:1 |
| BSA | 0 | .039 | .016 | .065 | .3-.5 | 4.6:1-7.7:1 |
| PPM | 2/0 | .039 | .016 | .065 | .3-.5 | 4.6:1-7.7:1 |
| BRD | 2/0 | .039 | .016 | .065 | .3-.5 | 4.6:1-7.7:1 |
| BRN | 2/0 | .039 | .016 | .065 | .3-.5 | 4.6:1-7.7:1 |
| BRS | 2/0 | .039 | .016 | .065 | .3-.5 | 4.6:1-7.7:1 |
| PPM | 0 | .044 | .016 | .065 | .3-.5 | 4.6:1-7.7:1 |
| PPM | 2/0 | .044 | .016 | .065 | .3-.5 | 4.6:1-7.7:1 |
| BRN | 4/0 | .020 | .010 | .055 | .3-.5 | 5.5:1-9.1:1 |
| BSA | 4/0 | .020 | .010 | .055 | .3-.5 | 5.5:1-9.1:1 |
| BRS | 4/0 | .020 | .010 | .055 | .3-.5 | 5.5:1-9.1:1 |
| BRS | 3/0 | .020 | .010 | .055 | .3-.5 | 5.5:1-9.1:1 |
| BRS | 2/0 | .020 | .010 | .055 | .3-.5 | 5.5:1-9.1:1 |
| BSA | 2/0 | .050 | .016 | .070 | .3-.5 | 4.3:1-7.1:1 |
| BSA | 0 | .050 | .016 | .070 | .3-.5 | 4.3:1-7.1:1 |
| SGC | 2/0 | .050 | .016 | .070 | .3-.5 | 4.3:1-7.1:1 |
| SGC | 0 | .050 | .016 | .070 | .3-.5 | 4.3:1-7.1:1 |
| BRN | 3/0 | .022 | .010 | .055 | .3-.5 | 5.5:1-9.1:1 |
| BRS | 3/0 | .022 | .010 | .055 | .3-.5 | 5.5:1-9.1:1 |
| BSA | 3/0 | .022 | .010 | .055 | .3-.5 | 5.5:1-9.1:1 |
| BRD | 0 | .050 | .016 | .065 | .3-.5 | 4.6:1-7.7:1 |
| BRD | 2/0 | .050 | .016 | .065 | .3-.5 | 4.6:1-7.7:1 |
| BRD | 0 | .050 | .016 | .065 | .3-.5 | 4.6:1-7.7:1 |
| BRS | 0 | .050 | .016 | .065 | .3-.5 | 4.6:1-7.7:1 |
| BRN | 0 | .050 | .016 | .065 | .3-.5 | 4.6:1-7.7:1 |
| PPM | 0 | .050 | .016 | .065 | .3-.5 | 4.6:1-7.7:1 |
| BRS | 2/0 | .050 | .016 | .065 | .3-.5 | 4.6:1-7.7:1 |
| BRN | 2/0 | .050 | .016 | .065 | .3-.5 | 4.6:1-7.7:1 |
| BRN | 3/0 | .039 | .010 | .055 | .3-.5 | 5.5:1-9.1:1 |
| SGC | 0 | .050 | .016 | .065 | .3-.5 | 4.6:1-7.7:1 |

TABLE I: NON-DETACHABLE SUTURES
(continued)

| Suture Type | Needle Size | Needle Diameter/ Inch | Shank Diameter/ Inch | EALS/ Inch | ALT/ Inch | Ratio of ALT to EALS |
|---|---|---|---|---|---|---|
| BRS | 0 | .060 | .016 | .065 | .3-.5 | 4.6:1-7.7:1 |
| PPM | 2/0 | .050 | .016 | .065 | .3-.5 | 4.6:1-7.7:1 |
| BRN | 0 | .050 | .016 | .065 | .3-.5 | 4.6:1-7.7:1 |
| BRN | 2/0 | .050 | .016 | .065 | .3-.5 | 4.6:1-7.7:1 |
| BRD | 2/0 | .050 | .016 | .065 | .3-.5 | 4.6:1-7.7:1 |
| PPM | 0 | .050 | .016 | .065 | .3-.5 | 4.6:1-7.7:1 |
| BRN | 5/0 | .020 | .010 | .055 | .3-.5 | 5.5:1-9.1:1 |
| BSA | 5/0 | .020 | .010 | .055 | .3-.5 | 5.5:1-9.1:1 |
| BRS | 5/0 | .020 | .010 | .055 | .3-.5 | 5.5:1-9.1:1 |
| BRS | 5/0 | .020 | .010 | .055 | .3-.5 | 5.5:1-9.1:1 |

TABLE II: DETACHABLE SUTURES

| Suture Type | Needle Size | Needle Diameter/ Inch | Shank Diameter/ Inch | EALS/ Inch | ALT/ Inch | Ratio of ALT to EALS |
|---|---|---|---|---|---|---|
| BSA | 3/0 | .024 | .010 | .055 | .095-.175 | 1.7:1-3.2:1 |
| BRD | 3/0 | .024 | .010 | .055 | .095-.175 | 1.7:1-3.2:1 |
| BRS | 3/0 | .024 | .010 | .055 | .095-.175 | 1.7:1-3.2:1 |
| BRN | 3/0 | .024 | .010 | .055 | .095-.175 | 1.7:1-3.2:1 |
| BRN | 3/0 | .032 | .010 | .055 | .095-.175 | 1.7:1-3.2:1 |
| BRS | 4/0 | .032 | .010 | .055 | .095-.175 | 1.7:1-3.2:1 |
| BRS | 3/0 | .032 | .010 | .055 | .095-.175 | 1.7:1-3.2:1 |
| BRN | 4/0 | .032 | .010 | .055 | .095-.175 | 1.7:1-3.2:1 |
| BSA | 2/0 | .026 | .016 | .065 | .095-.175 | 1.5:1-2.7:1 |
| BRD | 2/0 | .026 | .016 | .065 | .095-.175 | 1.5:1-2.7:1 |
| BRS | 2/0 | .026 | .016 | .065 | .095-.175 | 1.5:1-2.7:1 |
| BRN | 2/0 | .026 | .016 | .065 | .095-.175 | 1.5:1-2.7:1 |
| SGC | 0 | .044 | .016 | .065 | .095-.175 | 1.5:1-2.7:1 |
| BSA | 0 | .044 | .020 | .065 | .095-.175 | 1.5:1-2.7:1 |
| SGC | 1 | .044 | .020 | .070 | .095-.175 | 1.4:1-2.5:1 |
| BSA | 1 | .044 | .020 | .070 | .095-.175 | 1.4:1-2.5:1 |
| BRD | 1 | .050 | .020 | .070 | .095-.175 | 1.4:1-2.5:1 |
| BRN | 1 | .050 | .020 | .070 | .095-.175 | 1.4:1-2.5:1 |
| BSA | 1 | .050 | .020 | .070 | .095-.175 | 1.4:1-2.5:1 |
| BSA | 1 | .044 | .020 | .070 | .095-.175 | 1.4:1-2.5:1 |
| BRN | 1 | .039 | .020 | .070 | .095-.175 | 1.4:1-2.5:1 |
| SGC | 1 | .039 | .020 | .070 | .095-.175 | 1.4:1-2.5:1 |

## TABLE II: DETACHABLE SUTURES
### (continued)

| Suture Type | Needle Size | Needle Diameter/ Inch | Shank Diameter/ Inch | EALS/ Inch | ALT/ Inch | Ratio of ALT to EALS |
|---|---|---|---|---|---|---|
| BRS | 3/0 | .039 | .010 | .055 | .095-.175 | 1.7:1-3.2:1 |
| BSA | 3/0 | .039 | .010 | .055 | .095-.175 | 1.7:1-3.2:1 |
| PPM | 2/0 | .039 | .016 | .065 | .095-.175 | 1.5:1-2.7:1 |
| BRS | 0 | .039 | .016 | .065 | .095-.175 | 1.5:1-2.7:1 |
| BRD | 2/0 | .039 | .016 | .065 | .095-.175 | 1.5:1-2.7:1 |
| PPM | 0 | .039 | .016 | .065 | .095-.175 | 1.5:1-2.7:1 |
| BRN | 2/0 | .039 | .016 | .065 | .095-.175 | 1.5:1-2.7:1 |
| BRD | 0 | .039 | .016 | .065 | .095-.175 | 1.5:1-2.7:1 |
| BSA | 2/0 | .039 | .016 | .065 | .095-.175 | 1.5:1-2.7:1 |
| SGC | 0 | .039 | .016 | .065 | .095-.175 | 1.5:1-2.7:1 |
| BRN | 0 | .039 | .016 | .065 | .095-.175 | 1.5:1-2.7:1 |
| BSA | 0 | .039 | .020 | .065 | .095-.175 | 1.5:1-2.7:1 |
| BRS | 2/0 | .039 | .016 | .065 | .095-.175 | 1.5:1-2.7:1 |
| BSA | 0 | .050 | .020 | .065 | .095-.175 | 1.5:1-2.7:1 |
| BRD | 0 | .050 | .016 | .065 | .095-.175 | 1.5:1-2.7:1 |
| BRN | 0 | .050 | .016 | .065 | .095-.175 | 1.5:1-2.7:1 |
| PPM | 0 | .050 | .016 | .065 | .095-.175 | 1.5:1-2.7:1 |
| PPM | 0 | .039 | .016 | .065 | .095-.175 | 1.5:1-2.7:1 |
| BRD | 0 | .039 | .016 | .065 | .095-.175 | 1.5:1-2.7:1 |
| BRN | 0 | .039 | .016 | .065 | .095-.175 | 1.5:1-2.7:1 |
| BSA | 2/0 | .039 | .016 | .065 | .095-.175 | 1.5:1-2.7:1 |
| BSA | 0 | .039 | .020 | .065 | .095-.175 | 1.5:1-2.7:1 |
| PPM | 2/0 | .039 | .016 | .065 | .095-.175 | 1.5:1-2.7:1 |
| BRD | 2/0 | .039 | .016 | .065 | .095-.175 | 1.5:1-2.7:1 |
| BRN | 2/0 | .039 | .016 | .065 | .095-.175 | 1.5:1-2.7:1 |
| BRS | 2/0 | .039 | .016 | .065 | .095-.175 | 1.5:1-2.7:1 |
| PPM | 0 | .044 | .016 | .065 | .095-.175 | 1.5:1-2.7:1 |
| PPM | 2/0 | .044 | .016 | .065 | .095-.175 | 1.5:1-2.7:1 |
| BRN | 4/0 | .020 | .010 | .055 | .095-.175 | 1.7:1-3.2:1 |
| BSA | 4/0 | .020 | .010 | .055 | .095-.175 | 1.7:1-3.2:1 |
| BRS | 4/0 | .020 | .010 | .055 | .095-.175 | 1.7:1-3.2:1 |
| BRS | 3/0 | .020 | .010 | .055 | .095-.175 | 1.7:1-3.2:1 |
| BRS | 2/0 | .020 | .010 | .055 | .095-.175 | 1.7:1-3.2:1 |
| BSA | 2/0 | .050 | .016 | .070 | .095-.175 | 1.4:1-2.5:1 |
| BSA | 0 | .050 | .020 | .070 | .095-.175 | 1.4:1-2.5:1 |
| SGC | 2/0 | .050 | .016 | .070 | .095-.175 | 1.4:1-2.5:1 |

## TABLE II: DETACHABLE SUTURES
### (continued)

| Suture Type | Needle Size | Needle Diameter/ Inch | Shank Diameter/ Inch | EALS/ Inch | ALT/ Inch | Ratio of of ALT to EALS |
|---|---|---|---|---|---|---|
| SGC | 0 | .050 | .016 | .070 | .095-.175 | 1.4:1-2.5:1 |
| BRN | 3/0 | .022 | .010 | .055 | .095-.175 | 1.7:1-3.2:1 |
| BRS | 3/0 | .022 | .010 | .055 | .095-.175 | 1.7:1-3.2:1 |
| BSA | 3/0 | .022 | .010 | .055 | .095-.175 | 1.7:1-3.2:1 |
| BRD | 0 | .050 | .016 | .065 | .095-.175 | 1.5:1-2.7:1 |
| BRD | 2/0 | .050 | .016 | .065 | .095-.175 | 1.5:1-2.7:1 |
| BRD | 0 | .050 | .016 | .065 | .095-.175 | 1.5:1-2.7:1 |
| BRS | 0 | .050 | .016 | .065 | .095-.175 | 1.5:1-2.7:1 |
| BRN | 0 | .050 | .016 | .065 | .095-.175 | 1.5:1-2.7:1 |
| PPM | 0 | .050 | .016 | .065 | .095-.175 | 1.5:1-2.7:1 |
| BRS | 2/0 | .050 | .016 | .065 | .095-.175 | 1.5:1-2.7:1 |
| BRN | 2/0 | .050 | .016 | .065 | .095-.175 | 1.5:1-2.7:1 |
| BRN | 3/0 | .039 | .010 | .055 | .095-.175 | 1.7:1-3.2:1 |
| SGC | 0 | .050 | .016 | .065 | .095-.175 | 1.5:1-2.7:1 |
| BRS | 0 | .060 | .016 | .065 | .095-.175 | 1.5:1-2.7:1 |
| PPM | 2/0 | .050 | .016 | .065 | .095-.175 | 1.5:1-2.7:1 |
| BRN | 0 | .050 | .016 | .065 | .095-.175 | 1.5:1-2.7:1 |
| BRN | 2/0 | .050 | .016 | .065 | .095-.175 | 1.5:1-2.7:1 |
| BRD | 2/0 | .050 | .016 | .065 | .095-.175 | 1.5:1-2.7:1 |
| PPM | 0 | .050 | .016 | .065 | .095-.175 | 1.5:1-2.7:1 |
| BRN | 5/0 | .020 | .010 | .055 | .095-.175 | 1.7:1-3.2:1 |
| BSA | 5/0 | .020 | .010 | .055 | .095-.175 | 1.7:1-3.2:1 |
| BRS | 5/0 | .020 | .010 | .055 | .095-.175 | 1.7:1-3.2:1 |
| BRS | 5/0 | .020 | .010 | .055 | .095-.175 | 1.7:1-3.2:1 |

A gap may exist between shank 12 and suture 30 (as shown in Figs. 1 and 2) or shank 12 may abut against suture 30. As shown in Fig. 1, suture 30 may initially be of uniform cross-section throughout its length. Alternatively, the tip region of suture 30, i.e., the region inserted into tubing 20, may be of reduced cross-section relative to the remainder of suture 30, e.g., by tipping the suture tip with an adhesive or resinous tipping agent while suture 30 is under tension. (See, e.g., Canadian Patent No. 1,009,532 to Messores.) Reducing the diameter of the suture tip as by resin tipping may be desirable to prevent brooming of the suture, particularly for multifilament braided sutures, to rigidify the end of the suture to facilitate handling during attachment, and to allow a suture of larger diameter, e.g., a suture diameter equal to the diameter of the needle to which it is to be attached, to be more efficiently attached to the needle using the shrinkable tubing of the present invention. It is not necessary according to the present invention, however, to reduce the diameter of the tip region of suture 30 to efficiently attach needle 10 to suture 30. Indeed, it may be possible or desirable to apply a tipping agent to prevent brooming without reducing suture diameter. As shown in Fig. 1, shrinkable tubing 20 initially has an inner diameter that is larger than the outer diameter of the tip region of suture 30, thereby removing the importance or criticality of suture tipping.

After shrinkable tubing 20 is placed around shank 12 of needle 10 and the tip region of suture 30, energy is applied to tubing 20. In response to this energy, tubing 20 contracts or shrinks and engages shank 12 and suture 30. The overall length of tubing 20 may also be affected by the application of energy, e.g., the length of tubing 20 may reduce. Thus, the shrinking of tubing 20 brings the inner surface of tubing 20 into engagement with shank 12 and suture 30, thereby securing suture 30 to needle 10. Suitable energy sources include heat (convective or conductive), radiation, microwave energy, etc.

As shown in Figs. 1 and 2, shrinkable tubing 20 is simultaneously placed around both suture 30 and shank 12 of needle 10 in one embodiment of the present invention. It is preferable, however, to sequentially secure tubing 20 to needle 10 and suture 30. Thus, in a preferred embodiment of the present invention, shrinkable tubing 20 is initially secured to shank 12 through the localized application of energy to tubing 20 in the region surrounding shank 12. After tubing 20 has been brought into engagement with shank 12, suture 30 is inserted into tubing 20 and additional energy is applied thereto. Sequential shrinkage of tubing 20 makes it possible to vary the amount of energy used in securing tubing 20 to shank 12 and suture 30, respectively, and to limit the exposure of suture 30 to energy during the attachment process. It may also be desirable to cool suture 30 in the region outside tubing 20 to prevent any undesirable degradation thereof, e.g., with a cold air curtain.

As shown in Figs. 2 and 3, the shrinkage of tubing 20 typically compresses suture 30 to some extent. This is particularly true where the suture is a braided, multifilament material having void spaces in its structure. For example, tubing 20 may compress suture 30 by as much as 30 to 35% for a braided, synthetic absorbable suture and by a minimal amount for a relatively stiff material such as a monofilament surgical gut.

Shrinkable tubing 20 can be manufactured from any material which shrinks, i.e., reduces in diameter, in response to the application of energy. Suitable materials for tubing 20 include "memory metals", e.g., nickel-titanium mixtures, nickel-iron-titanium mixtures, or copper based materials, as are well known in the art (see, e.g., U.S. Patent Nos. 3,759,552, 3,801,954, 4,198,081 and 4,773,680), and shrinkable plastic materials such as polyvinylidene fluoride materials available from Raychem Corporation, Menlo Park, California, under the tradename Kynar. If desired, the plastic materials can be provided with fibrous reinforcements to increase their tensile strength. Tubing 20 is typically extruded in such a manner that the inner diameter will be less than the final desired inner diameter, i.e., the inner diameter of the tubing after energy application in the attachment method of the present invention. Thereafter, the extruded tubing is expanded radially outward through radial expansion means to provide a tubing of expanded inner diameter as shown, for example, by tubing 20 in Figure 1. Such plastic tubing is thus adapted to shrink or "recover" to its original extruded inner diameter in response to the application of a predetermined amount of energy.

The amount of energy applied to tubing 20 to effect the desired attachment, i.e., diameter reduction, depends upon the chemical characteristics of the tubing material, the relative dimensions of the tubing, the shank end of the needle and the suture, and the desired pull-out force for the needle-suture combination. For example, one polyvinylidene fluoride material available from Raychem Corporation (RT-850) shrinks at temperatures greater than 175°C, and is adapted to recover to about 50% of its radially expanded inner diameter. In such case, tubing 20 may be brought into engagement with shank 12 of needle 10 and suture 30, either simultaneously or sequentially, by heating tubing 20 to a temperature above 175°C. Tubing 20 may be heated through contact with a hot gas stream or with heated dies, or by other heating means. Typically, the outer diameters of shank 12 and suture 30 (in the region inserted into tubing 20) are greater than the fully recovered diameter of tubing 20, e.g., greater than 50% of the initial inner diameter of tubing 20 for the RT-850 material, such that tubing 20 engages shank 12 and suture 30. This engagement provides the needle-suture combination of the present invention.

As noted above, the attachment method of the present invention may be easily used to effect both standard needle attachment and detachable needle attachment. Preferably, the pull-out force of a given needle-suture combination is controlled by regulating the ratio of the axial length of the tubing to the effective axial length of the shank. Thus, using the identical inventories of needles, sutures and tubings, it is possible to produce either standard or detachable needle products through simple energy variations. In the case of detachable needle attachment, it is preferred that the attachment conditions be controlled such that the tubing remains secured to the needle once the suture is detached.

The needle-suture attachment method of the present invention has many advantages over previous attachment methods. Machining of the needle to provide a reduced diameter needle shank is much easier and more controllable than drilling processes, and permits the use of needle alloys which have previously been impractical, e.g., Series 300 stainless steel and MP35N (available from SPS Technologies). These heretofore impractical alloys have advantageous strength and ductility characteristics as compared to conventionally used Series 400 stainless steels. Moreover, an unreliable, expensive and maintenance intensive swaging process is replaced by a sterile, controllable and relatively inexpensive energy supply. The tubing used in the present invention may be color coded to designate suture material, standard versus detachable attachment, etc., particularly where a plastic tubing is employed.

The attachment method of the present invention is also much more efficient from a processing and inventory control standpoint. For example, the tubing may be removed from a needle and the needle attached to a fresh suture, e.g., in instances where the suture and/or attachment properties of the initial suture-needle combination are outside specifications. In many instances, the suture may also be recovered and reused, thereby greatly reducing processing waste. The range of acceptable suture diameters is greatly expanded due to the ability of the tubing to recover or shrink to varying degrees, thereby minimizing the likelihood that suture production will be rejected for inability to attach several needle sizes because the shrinkable tubing is capable of recovering or shrinking to varying degrees. This greatly simplifies inventory considerations.

Needle-suture combinations produced according to the present invention are atraumatic and advantageously exhibit flexibility in the attachment region. Both standard needle attachment and detachable needle attachment products may be produced with great processing ease.

While the above description contains many specifics, these specifics should not be construed as limitations on the scope of the invention, but merely as exemplifications of preferred embodiments thereof. Those skilled in the art will envision many other possible variations that are within the scope and spirit of the invention as defined by the claims appended hereto.

## Claims

1. A combined surgical needle-suture device which comprises:
   a) a needle having a shank of reduced cross-section;
   b) a suture; and,
   c) a shrinkable tubing around said needle shank and a portion of said suture.

2. The combined surgical needle-suture device of Claim 1 wherein the shrinkable tubing is manufactured from a memory metal or a shrinkable plastic material.

3. The combined surgical needle-suture device of Claim 1 characterized in that the pull-out force for said combination is as defined for standard needle attachment.

4. The combined surgical needle-suture device of Claim 1 characterized in that the pull-out force for said combination is as defined for removable or detachable needle attachment.

5. The combined surgical needle-suture device of Claim 1 wherein the butt end of the shank of the needle is rounded.

6. The combined surgical needle-suture device of Claim 1 wherein the ratio of the axial length of the shrinkable tubing to the effective axial length of the needle shank is at least about 4:1 when the needle is intended to be non-detachable from the suture and no more than about 3.5:1 when the needle is intended to be detachable from the suture.

7. A method for attaching a surgical needle to a suture which comprises:
   a) providing a needle having a shank of reduced cross-section;
   b) placing a shrinkable tubing around the reduced diameter shank and the suture; and,
   c) applying energy to the shrinkable tubing to bring the tubing into engagement with the needle shank and the suture.

8. The method of Claim 7 wherein said shrinkable tubing is manufactured from a memory metal or a shrinkable plastic material.

9. The method of Claim 7 wherein the butt end of the needle shank is rounded.

10. The method of Claim 7 wherein the axial length of the tubing to the effective axial length of the needle shank is at least about 4:1 when the needle is intended to be non-detachable from the suture and no more than about 3.5:1 when the needle is intended to be detachable from the suture.

FIG. 1

FIG. 2

FIG. 3

# EUROPEAN SEARCH REPORT

**EP 90 31 0521**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,Y | FR-A-2 268 534 (ALBANY) <br> * Figure 4; page 8, last paragraph * <br> — — — | 1-10 | A 61 B 17/06 |
| D,Y | US-A-1 613 206 (SOUTTAR) <br> * Figure 2; claim 1; page 1, lines 47-50 * <br> — — — | 1-10 | |
| A | CH-B-5 122 37 (SUTRAMEO) <br> * Figure 3; sub-claim 2 * <br> — — — | 2,7 | |
| D,A | US-A-3 981 307 (BORYSKO) <br> * Abstract; figure 2; column 5, lines 38-40 * <br> — — — | 5 | |
| A | DE-A-3 223 153 (MELSUNGEN) <br> — — — — — | | |

|  |  |  |  |
|---|---|---|---|
| | | | **TECHNICAL FIELDS SEARCHED (Int. Cl.5)** |
| | | | A 61 B |

The present search report has been drawn up for all claims

| Place of search | Date of completion of search | Examiner |
|---|---|---|
| The Hague | 17 December 90 | BARTON S.A. |

CATEGORY OF CITED DOCUMENTS

X : particularly relevant if taken alone
Y : particularly relevant if combined with another document of the same catagory
A : technological background
O : non-written disclosure
P : intermediate document
T : theory or principle underlying the invention

E : earlier patent document, but published on, or after the filing date
D : document cited in the application
L : document cited for other reasons

&: member of the same patent family, corresponding document